# EUROPÄISCHE PATENTSCHRIFT

(11) **EP 0 760 091 B1**
(45) Veröffentlichungstag und Bekanntmachung des Hinweises auf die Patenterteilung: **03.11.1999**
(21) Anmeldenummer: 95917276.8
(22) Anmeldetag: 03.05.1995
(51) Int. Cl.: G01N 21/47, A61B 5/00

(54) **VERFAHREN UND VORRICHTUNG ZUR BESTIMMUNG EINES ANALYTEN IN EINER BIOLOGISCHEN PROBE**
PROCESS AND DEVICE FOR DETERMINING AN ANALYTE IN A BIOLOGICAL SAMPLE
PROCEDE ET DISPOSITIF POUR LA DETERMINATION D'UN ANALYTE DANS UN ECHANTILLON BIOLOGIQUE

(30) Priorität: 19.05.1994 DE 4417639; 29.10.1994 WO PCT/DE94/01290
(43) Veröffentlichungstag der Anmeldung: 05.03.1997
(73) Patentinhaber: Roche Diagnostics GmbH, 68298 Mannheim (DE)
(72) Erfinder: ESSENPREIS, Matthias, D-82131 Gauting (DE); BOECKER, Dirk, D-69115 Heidelberg (DE); HEIN, Heinz-Michael, D-64342 Seeheim-Jugenheim (DE); HAAR, Hans-Peter, D-69168 Wiesloch (DE)
(74) Vertreter: Pfeifer, Hans-Peter, Dr., Dr. H.-P. Pfeifer Dr. P. Jany, Patentanwälte
(86) Internationale Anmeldenummer: DE9500593
(87) Internationale Veröffentlichungsnummer: WO9532416

(56) Entgegenhaltungen:
- EP-A- 0 580 414
- EP-A- 0 592 200
- EP-A- 0 627 619
- EP-A- 0 627 620
- EP-A- 0 663 591
- WO-A-89/12223
- WO-A-90/09003
- WO-A-93/11701
- SPIE PROCCEEDINGS, Bd.1888, September 1993, ISSN 0-8194-1115 Seiten 248 - 257 A. DUNCAN ET AL. 'a multiwavelength, wideband, intensity modulated optical spectrometer for infrared spectroscopy and imaging' in der Anmeldung erwähnt

## Beschreibung

Die Erfindung betrifft ein Verfahren und eine Vorrichtung zur Bestimmung der Konzentration eines Analyten in einer biologischen Probe.

Der Begriff "biologische Probe" bezeichnet eine Körperflüssigkeit oder ein Gewebe eines lebenden Organismus. Biologische Proben sind meist optisch heterogen, d.h. sie enthalten eine Vielzahl von Streuzentren, an denen eingestrahltes Licht gestreut wird. Im Falle von biologischem Gewebe, insbesondere Hautgewebe, werden die Streuzentren von den Zellwänden und anderen in dem Gewebe enthaltenen optisch heterogenen Bestandteilen gebildet.

Körperflüssigkeiten, insbesondere Blut, sind ebenfalls optisch heterogene biologische Proben, weil sie Partikel enthalten, an denen die Primärstrahlung gestreut wird. Auch Milch und andere in der Lebensmittelchemie zu untersuchende Flüssigkeiten enthalten vielfach eine hohe Konzentration von Streuzentren, beispielsweise in Form von emulgierten Fetttröpfchen.

Zur qualitativen und quantitativen analytischen Bestimmung von Komponenten solcher biologischen Proben werden im allgemeinen Reagenzien bzw. Reagenziensysteme eingesetzt, die mit der jeweiligen Komponente chemisch reagieren. Die Reaktion führt zu einer physikalisch nachweisbaren Änderung der Reaktionslösung, beispielsweise einer Änderung ihrer Farbe, die als Meßgröße gemessen werden kann. Durch Kalibration mit Standardproben bekannter Konzentration wird eine Korrelation zwischen den bei unterschiedlichen Konzentrationen gemessenen Werten der Meßgröße und der jeweiligen Konzentration bestimmt. Diese Verfahren ermöglichen Analysen mit hoher Genauigkeit und Empfindlichkeit, machen es jedoch erforderlich, eine flüssige Probe, insbesondere eine Blutprobe, zur Analyse dem Körper zu entnehmen ("Invasive Analyse"). Diese Probenentnahme ist unangenehm und schmerzhaft und führt zu einem gewissen Infektionsrisiko.

Es sind deshalb bereits eine Vielzahl von Verfahren und Vorrichtungen vorgeschlagen worden, um Analyten in Blut, Gewebe oder anderen biologischen Proben in vivo und nicht-invasiv zu bestimmen.

Die Erfindung bezieht sich auf eine Teilgruppe solcher Verfahren, bei denen Meßlicht mittels Einstrahlungsmitteln durch eine die Probe begrenzende Grenzfläche als Primärlicht an einem Einstrahlungsort in diese eingestrahlt und aus der biologischen Probe durch eine diese begrenzende Grenzfläche in einem vorbestimmten Meßabstand an einem Detektionsort austretendes Licht mittels Detektionsmitteln detektiert wird, um eine durch die Wechselwirkung mit der biologischen Probe (ohne Verwendung von Reagenzien) veränderliche physikalische Eigenschaft des Lichts zu bestimmen, die mit der Konzentration des Analyten in der biologischen Probe korreliert. Ein solcher Verfahrensschritt wird nachfolgend als "Detektionsschritt" bezeichnet.

Die in einem Detektionsschritt bestimmte (detektierte) mit der Analytkonzentration korrelierende physikalische Eigenschaft des Lichtes, die man auch als "quantifizierbarer Parameter" (englisch: quantifiable parameter) bezeichnen kann, wird nachfolgend einfachheitshalber als "Meßgröße" bezeichnet. Dieser Begriff darf aber nicht dahingehend verstanden werden, daß ein bestimmter Betrag der Meßgröße in einer entsprechenden Maßeinheit gemessen werden muß.

Soweit die Verfahren keine Absolutmessung ermöglichen, ist (ebenso wie bei den gebräuchlichen, auf chemischen Reaktionen basierenden Analyseverfahren) eine Kalibration erforderlich. Üblicherweise wird in mindestens einem Kalibrationsschritt, der meßtechnisch in gleicher Weise wie der Detektionsschritt ausgeführt wird, die Meßgröße an einer biologischen Probe mit bekannter Analytkonzentration bestimmt.

In einem Auswerteschritt des Analyseverfahrens wird die Analytkonzentration aus der bei mindestens einem Detektionsschritt gemessenen Meßgröße, gegebenenfalls im Vergleich zu mindestens einem Kalibrationsschritt, ermittelt. Der Auswerteschritt umfaßt einen Auswertealgorithmus, in dem die Konzentration in vorbestimmter Weise aus den Ergebnissen von mindestens einem Detektionsschritt und in der Regel mindestens einem Kalibrationsschritt berechnet wird.

Die Wellenlängen des Lichts, die für solche Verfahren diskutiert werden, liegen allgemein zwischen etwa 300 nm und mehreren tausend nm, also im Spektralbereich zwischen dem nahen UV- und infrarotem Licht. Der Begriff "Licht" darf nicht als Einschränkung auf den sichtbaren Spektralbereich des Lichtes verstanden werden.

Der Erfindung liegt die Aufgabe zugrunde, ein Verfahren für die Konzentrationsbestimmung von Analyten in einer biologischen Probe zur Verfügung zu stellen, welches reagenzienfrei und nicht-invasiv arbeitet und eine gute Analysegenauigkeit, zum Beispiel für die Beobachtung der Änderung der Analytkonzentration (Verlaufskontrolle) über einen ausreichenden Zeitraum, ermöglicht.

Die Aufgabe wird gelöst durch ein Verfahren zur analytischen Bestimmung der Konzentration eines Analyten in einer biologischen Probe, bei welchem in einem Detektionsschritt Licht mittels Einstrahlungsmitteln von einem Lichtsender durch eine die biologische Probe begrenzende Grenzfläche als Primärlicht an einem Einstrahlungsort in die Probe eingestrahlt und aus der biologischen Probe durch eine diese begrenzende Grenzfläche an einem Detektionsort in einem vorbestimmten Meßabstand von dem Einstrahlungsort austretendes Licht mittels Detektionsmitteln als Sekundärlicht detektiert wird, um eine durch die Wechselwirkung mit der biologischen Probe veränderliche, meßbare physikalische Eigenschaft des Lichtes als Meßgröße zu bestimmen, die mit der Konzentration des Analyten in der biologischen Probe korreliert und in einem Auswerteschritt die Analytkonzentration auf Basis der in dem Detektionsschritt gemessenen Meßgröße ermittelt wird, welches dadurch gekennzeichnet ist, daß in dem Detektionsschritt (a) ohne Bewegung der Lichteinstrahlungsmittel oder der Detektionsmittel Frequenzdomänen-spektroskopische Messungen mit mindestens zwei unterschiedlichen Meßlichtwegen zwischen Einstrahlungsort und Detektionsort durchgeführt werden, (b) jede der Frequenzdomänen-spektroskopischen Messungen bei mindestens zwei unterschiedlichen Lichtwellenlängen durchgeführt wird, und (c) bei den Frequenzdomänen-spektroskopischen Messungen jeweils die Phasenverschiebung des Sekundärlichts gegenüber dem Primärlicht sowie eine Intensitätsmeßgröße bestimmt wird.

Die Intensitätsmeßgröße ist vorzugsweise entweder die DC-Intensitätskomponente des Sekundärlichts oder die AC-Intensitätskomponente des Sekundärlichts. Die biologische Probe ist insbesondere ein biologisches Gewebe, speziell ein Hautgewebe, insbesondere der Fingerbeere, der Oberbauchdecke, des Nagelbetts, der Lippe, der Zunge oder des inneren Oberarms des Menschen oder Muskelgewebe oder subcutanes Fettgewebe oder das Gewebe der Skleren.

Frequenzdomänen-spektroskopische Meßverfahren (frequency domain spectrometry) zeichnen sich dadurch aus, daß das Primärlicht in seiner Intensität mit einer hochfrequenten Modulationsfrequenz (typischerweise zwischen 50 MHz und einigen GHz) moduliert wird. Dabei wird als Meßgröße nicht - wie bei der klassischen Spektroskopie - ausschließlich die statische Intensität des Sekundärlichts, sondern mindestens eine dynamische Meßgröße, insbesondere die Phasenverschiebung und die AC-Amplitude des modulierten Sekundärlichts im Vergleich zu dem Primärlicht gemessen.

Gebräuchlich sind Frequenzdomänen-spektroskopische Verfahren insbesondere zur Bestimmung der Lebensdauer von fluoreszierenden Farbstoffen bei analytischen Verfahren, die darauf basieren, den Analyt durch eine spezifische Bindungsreaktion mit einer fluoreszierenden Markierung zu versehen. Bei einem Teil solcher Verfahren wird die Fluoreszenz-Lebensdauer als Meßgröße bestimmt ("Phasenfluorometrie"). Um die sehr kurzen Lebensdauern (in der Größenordnung von weniger als 100 psec.) bestimmen zu können, wurden Radiofrequenz (RF, radio frequency)-modulierte Spektrometer entwickelt, die auf Basis einer Intensitätsmodulation des Lichtes mit einer Frequenz zwischen etwa 50 MHz und mehreren GHz arbeiten.

Ein wichtiges Beispiel eines Meßverfahrens nach diesem Prinzip ist das Heterodyn-Verfahren, bei dem das von dem Lichtempfänger (Detektor) empfangene Signal mit einem zweiten periodischen Signal gemischt wird, dessen Frequenz sich von dem ersten Signal um einen kleinen Bruchteil von dessen Modulationsfrequenz (z.B. wenige kHz) unterscheidet. Das resultierende Differenzsignal kann verhältnismäßig einfach mit einem Lock-in-Verstärker oder sonstigen schmalbandig selektierenden Verstärkungsverfahren aufbereitet und gemessen werden. Eine leistungsfähige Apparatur für solche Messungen ist beschrieben in J.R. Lakowicz et al.: "2-GHz frequency-domain fluorometer", Rev.Sci.Instrum., 57 (1986), 2499-2506. Ein Verfahren zur Analyse von Glucose mit Hilfe der Phasenmodulations-Flourometrie ist aus J.R. Lakowicz und B. Maliwal: "Optical sensing of glucose using phase-modulation fluorometry", Analytica Chimica Acta, 271, (1993) 155-164 bekannt. Es handelt sich dabei um einen In-vitro-Test, bei dem die Probe in konventioneller Weise invasiv entnommen und mit den erforderlichen Reagenzien, die eine fluoreszierende Markierung enthalten, gemischt werden muß.

Eine neue apparative Entwicklung auf diesem Gebiet ist beschrieben in: A. Duncan et al. "A multiwavelength, wide band, intensity modulated optical spectrometer for near infrared spectroscopy and imaging", Proc. SPIE 1888 (1993), 248-257.

In den vorstehenden Publikationen sind zahlreiche meßtechnische Einzelheiten beschrieben, die unter Berücksichtigung der nachfolgenden Erläuterungen auch bei der vorliegenden Erfindung vorteilhaft einsetzbar sind.

Die Frequenzdomänen-spektroskopischen Messungen werden mit mehreren unterschiedlichen Meßlichtwegen innerhalb der Probe zwischen Einstrahlungsort und Detektionsort durchgeführt. Dabei ist wesentlich, daß die Einstellung der unterschiedlichen Meßlichtwege bewegungslos, insbesondere ohne Bewegung der Lichteinstrahlungsmittel oder der Detektionsmittel, erfolgt. Es wurde gefunden, daß die Möglichkeit einer hinreichend genauen Bestimmung des Absorptionsparameters und des Streuparameters in biologischen Proben, insbesondere Hautgewebe wesentlich davon abhängt, daß die Variation des Abstandes zwischen Einstrahlungsort und Detektionsort nicht durch Bewegen der Einstrahlungsmittel und/oder der Detektionsmittel realisiert wird.

Im Rahmen der Erfindung werden in dem Detektionsschritt (dessen Meßergebnisse in dem Auswerteschritt zu dem gewünschten Analyseergebnis weiterverarbeitet werden) mindestens acht Meßwerte (Phasendifferenz und Intensitätsmeßwert jeweils für mindestens zwei Meßlichtwege und zwei Lichtwellenlängen) bestimmt. Daraus wird das analytische Ergebnis vorzugsweise nicht unmittelbar ermittelt, sondern aus den mindestens acht Meßwerten werden getrennte Parameter bestimmt, von denen einer ein Maß der Streuung des Lichts (Streuparameter) und der andere ein Maß der optischen Absorption des Lichts (Absorptionsparameter) in der biologischen Probe ist. Unter einem Streuparameter in diesem Sinne ist in erster Linie der Streukoeffizient und unter einem Absorptionsparameter in erster Linie der optische Absorptionskoeffizient zu verstehen. Es ist allerdings im Rahmen der Erfindung nicht erforderlich, diese Parameter quantitativ in den gebräuchlichen Maßeinheiten zu berechnen. Vielmehr genügt es, wenn reproduzierbar zwei unabhängige Parameter ermittelt werden, die in dem Sinne voneinander unabhängig sind, daß der Streuparameter nur für die Streuung in der Probe charakteristisch (d.h. von der optischen Absorption der Probe unabhängig) und der Absorptionsparameter nur von der optischen Absorption in der Probe abhängig (d.h. von der Streuung unabhängig) ist.

Geeignete Verfahren zur getrennten Bestimmung des Streukoeffizienten und des Absorptionskoeffizienten in einer optisch heterogenen Matrix werden in der vorpublizierten Literatur beschrieben, insbesondere in:
M.S. Patterson et al., "Frequency-domain reflectance for the determination of the scattering and absorption properties of tissue", APPLIED OPTICS, 30, pp 4474 - 4476 (1991) und
Gratton et al., "Near-infrared optical spectroscopy of tissues using an LED frequency domain spectrometer," Advances in Optical Imaging and Photon Migration, Technical Digest, 1994, pp 212-215.

Diesen Publikationen und den darin beschriebenen Verfahren zur Berechnung des Absorptionskoeffizienten und des Streukoeffizienten liegt eine bestimmte mathematisch-physikalische Vorstellung, nämlich die Diffusionstheorie, zugrunde. Es ist jedoch auch möglich, den Auswerteschritt, bei dem aus den mindestens acht Meßwerten die Analytkonzentration abgeleitet wird, rein empirisch mit Hilfe eines mathematischen Verknüpfungsalgorithmus durchzuführen. Es sind geeignete Verfahren der numerischen Mathematik verfügbar, mit denen die Meßwerte als Eingangsvariablen mit dem analytischen Ergebnis als Ausgangsvariablen verknüpft werden können. Hierzu gehören iterative Methoden zur optimalen Beschreibung der Relation von Eingangsvariablen und Ausgangsvariablen. Multilineare und nichtlineare Algorithmen können verwendet werden, um mehrere Faktoren miteinander zu verknüpfen und einer einzigen oder einer geringeren Anzahl von Ausgangsvariablen zuzuordnen. Dabei besteht die Möglichkeit, zugleich andere Einflußfaktoren (wie beispielsweise die Temperatur am Meßort) zusätzlich zu berücksichtigen.

Hierzu ist ein Lernprozeß auf Basis einer Kalibration erforderlich, wobei gleichartige Detektionsschritte an biologischen Proben mit bekannter Analytkonzentration durchgeführt werden. Der dabei gewonnene Datensatz (Meßwerte der Meßgrößen in Relation zu der Analytkonzentration) wird zur Ermittlung eines mathematischen Algorithmus verwendet, mit dem die Meßwerte (Eingangsvariablen) mit den Analyseergebnissen (Ausgangsvariablen) verknüpft werden. Nach erfolgter Kalibration lassen sich aus den Meßwerten unter Verwendung dieses Algorithmus die Analyseergebnisse ermitteln. Geeignete Verfahren, beispielsweise auch in Form neuronaler Netze, sind bekannt und als Computerprogramme kommerziell erhältlich.

Die bewegungslose Einstellung der unterschiedlichen Meßlichtwege wird gemäß einer ersten bevorzugten Ausführungsform dadurch realisiert, daß mit mehreren Detektionsmitteln jeweils an unterschiedlichen Detektionsorten austretendes Licht erfaßt wird, wobei die Detektionsorte unterschiedliche Meßabstände zu mindestens einem Einstrahlungsort haben. Dabei gibt es zwei Möglichkeiten.

Zum einen kann das an mehreren unterschiedlichen Detektionsorten austretende Licht über lichtleitende optische Elemente (insbesondere Lichtleitfasern) zu einem gemeinsamen Lichtempfänger geleitet werden. Die Umschaltung erfolgt dabei bevorzugt mit Hilfe eines optischen Schalters.

Zum zweiten können mehrere Lichtempfänger verwendet werden, die das an den unterschiedlichen Detektionsorten austretendes Sekundärlicht getrennt detektieren, wobei diese Lichtempfänger entweder unmittelbar an dem Detektionsort angeordnet oder wiederum über lichtleitende Elemente mit diesem verbunden sein können. Diese Möglichkeit, die nachfolgend als PDOLS (plural detector one light source) bezeichnet wird, erscheint zunächst nachteilig, weil die von jedem Lichtempfänger erzeugten Signale getrennt verstärkt und weiterverarbeitet werden müssen, während bei der Realisierung mehrerer unterschiedlicher Meßabstände mit nur einem Lichtempfänger und mehreren zugeordneten Lichtsendern, deren Licht an mehreren unterschiedlichen Einstrahlungsorten eingestrahlt wird (nachfolgend als PLSOD für "plural light source one detector" bezeichnet), lediglich die Mehrzahl von Lichtsendern nacheinander angesteuert werden müssen, was elektronisch wesentlich weniger aufwendig erscheint.

Im Rahmen der vorliegenden Erfindung wurde gefunden, daß es - insbesondere unter Berücksichtigung der hier beschriebenen besonderen Maßnahmen - nicht nur möglich ist, mit vertretbarem meßtechnischem Aufwand eine Meßvorrichtung nach dem PDOLS-Prinzip zu realisieren, sondern daß dadurch besondere Vorteile hinsichtlich der Bestimmung von mit einem Analyten korrelierenden Meßgrößen in biologischen Proben erzielt werden. Insbesondere erweist es sich als sehr vorteilhaft, daß die zeitliche Variation der gewebeoptischen Parameter (Absorptionsparameter und Streuparameter) simultan an mehreren Meßorten erfaßt wird. Das PLSOD-Prinzip wird dagegen aufgrund seiner sequentiellen Meßweise zusätzlich zu der unvermeidlichen örtlichen Variation des Gewebes auch durch dessen zeitliche Variation beeinflußt. Diese zeitlichen Fluktuationen können so schnell sein, daß es kaum möglich ist, sie bei einer PLSOD-Messung durch rasches Umschalten der Lichtsender (innerhalb der meßelektronisch vorgegebenen Grenzen) zu eliminieren.

Gemäß einer zweiten bevorzugten Ausführungsform wird die bewegungslose Einstellung der unterschiedlichen Meßlichtwege dadurch realisiert, daß bei gleichem Meßabstand zwischen Einstrahlungsort und Detektionsort das Primärlicht mit unterschiedlichen Modulationsfrequenzen moduliert wird. Die Variation der Modulationsfrequenz führt - wie weiter unten noch näher erläutert wird - zu Änderungen des Meßlichtweges in der Probe, die alternativ oder zusätzlich zu Änderungen des Meßabstandes verwendet werden können, um bewegungslos unterschiedliche Meßlichtwege einzustellen.

In dem Detektionsschritt werden mindestens zwei Frequenzdomänen-spektroskopische Messungen mit jeweils mindestens zwei unterschiedlichen Wellenlängen des Primärlichts durchgeführt. Insoweit ähnelt das Verfahren klassischen spektroskopischen Verfahren, insbesondere der "Zwei-Wellenlängen-Spektroskopie", bei der eine erste Meßwellenlänge so gewählt ist, daß der Analyt möglichst stark absorbiert, während eine zweite Wellenlänge als Referenzwellenlänge so gewählt ist, daß die Lichtabsorption möglichst wenig von der Analytkonzentration abhängt. Selbstverständlich können auch mehrere Wellenlängen für die einzelnen Detektionsschritte verwendet werden, wobei das Spektrum in bekannter Weise, zum Beispiel mittels Verfahren der multivariaten Analyse, ausgewertet wird. Dabei besteht auch die Möglichkeit, mehrere Analyten zu bestimmen. Wichtige Analyten sind insbesondere Glucose, Bilirubin, Cholesterin, Triglyceride, Harnstoff, Harnsäure, Oxihämoglobin, Hämoglobin und Cytochromoxidase.

Gemäß einer weiteren bevorzugten Ausführungsform kann die Erfindung verwendet werden, um die Änderung der Konzentration eines Analyten zu beobachten, der zuvor dem untersuchten Gewebe als exogene Substanz zugeführt worden ist. Beispielsweise kann ein mit der Erfindung als Analyt nachweisbarer Farbstoff intravenös zugeführt werden. Auch eine Substanz, die den Streuparameter beeinflußt, kann hierfür vorteilhaft verwendet werden. Dies kann vorteilhaft beispielsweise bei sogenannten Clearance-Tests (Funktionstests von Organen) oder zur Therapiekontrolle (Überwachung der Einlagerung und Metabolisierung von Therapeutika im Körper) eingesetzt werden. Die exogene Substanz kann auch dazu dienen, pathologische Strukturen im Gewebe durch Kontrastverstärkung zu detektieren.

Die Konzentration von Glucose als Analyt wird vorzugsweise aus dem Streuparameter abgeleitet. In der PCT-Anmeldung PCT/DE 93/01058 wird der Zusammenhang zwischen der Konzentration von Glucose und der Streuung in einer optisch heterogenen biologischen Probe näher erläutert. Die Trennung der Einflüsse der optischen Absorption und der Streuung im Rahmen der vorliegenden Erfindung ermöglicht die Absolutmessung eines Streuparameters und damit die absolute Messung von Änderungen der Glucosekonzentration. Durch die Abtrennung des Absorptionseinflusses sind Störungen durch stark absorbierende Substanzen in der Probe, wie beispielsweise Hämoglobin, vernachlässigbar. Deshalb ist auf Basis eines Streuparameters, der von den Einflüssen der Absorption in der Probe befreit ist, eine genauere Bestimmung der Glucosekonzentration möglich.

Gemäß einem zweiten Hauptaspekt, der vorzugsweise in Verbindung mit dem ersten Hauptaspekt (soweit er sich auf Gewebe bezieht) zur Anwendung kommt, jedoch auch selbständige Bedeutung hat, richtet sich die Erfindung auf ein Verfahren zur analytischen Bestimmung der Konzentration eines Analyten in einem biologischen Gewebe, bei welchem in einem Detektionsschritt Licht von einem Lichtsender durch eine die biologische Probe begrenzende Grenzfläche als Primärlicht in das Gewebe eingestrahlt und aus dem biologischen Gewebe durch eine diese begrenzende Grenzfläche in einem vorbestimmten Meßabstand austretendes Licht von einem Lichtempfänger als Sekundärlicht detektiert wird, um eine durch die Wechselwirkung mit der biologischen Probe veränderliche, meßbare physikalische Eigenschaft des Lichtes als Meßgröße zu bestimmen, die mit der Konzentration des Analyten in dem Gewebe korreliert und in einem Auswerteschritt die Analytkonzentration auf Basis der in dem Detektionsschritt gemessenen Meßgröße ermittelt wird, welches dadurch gekennzeichnet ist, daß der Lichtweg in dem Gewebe auf ein vorbestimmtes Teilvolumen des Gewebes eingestellt wird, in dem die Analytkonzentration bestimmt werden soll.

Der Lichtweg in der biologischen Probe kann durch geeignete Wahl von Meßabstand und Modulationsfrequenz in definierter Weise auf ein vorbestimmtes Teilvolumen eingestellt werden, wie weiter unter noch näher erläutert wird.

Die Erfindung wird nachfolgend anhand von in den Figuren schematisch dargestellten Ausführungsbeispielen näher erläutert. Es zeigen:
- Fig. 1: Ein Blockschaltbild einer für die Erfindung geeigneten Meß- und Auswertevorrichtung,
- Fig. 2: ein Blockschaltbild einer weiteren Ausführungsform einer für die Erfindung geeigneten Meß- und Auswertevorrichtung,
- Fig. 3: einen Ausschnitt aus einer Schaltung für eine Meßvorrichtung nach dem PDOLS-Prinzip,
- Fig. 4: eine Aufsicht auf die Leiterplatte einer Meß- vorrichtung nach dem PDOLS-Prinzip,
- Fig. 5: eine Seitenansicht einer Leiterplatte von Fig. 4 und einer Kontaktplatte,
- Fig. 6: eine Explosionsdarstellung einer alternativen Ausführungsform der Kontaktplatte eines für die Erfindung geeigneten Meßkopfes,
- Fig. 7: einen Ausschnitt aus einer Schaltung zur Darstellung einer bevorzugten Ausführungsform der Frequenzstabilisierung für zwei Frequenzgeneratoren zur Erzeugung einer konstanten Differenz-frequenz,
- Fig. 8: den effektiven Lichtweg in einem biologischen Gewebe bei zwei unterschiedlichen Modulationsfrequenzen,
- Fig. 9: den effektiven Lichtweg in einem biologischen Gewebe bei zwei unterschiedlichen Meßabständen und gleicher Modulationsfrequenz,
- Fig. 10: den effektiven Lichtweg in einem biologischen Gewebe bei unterschiedlichen Meßabständen und auf maximale Überlappung optimierten Modulationsfrequenzen,
- Fig. 11: den optischen Lichtweg in einem biologischen Gewebe bei Verwendung einer zwischen der Einstrahlungsstelle und der Detektionsstelle auf der Gewebeoberfläche aufliegenden reflektierenden Fläche.

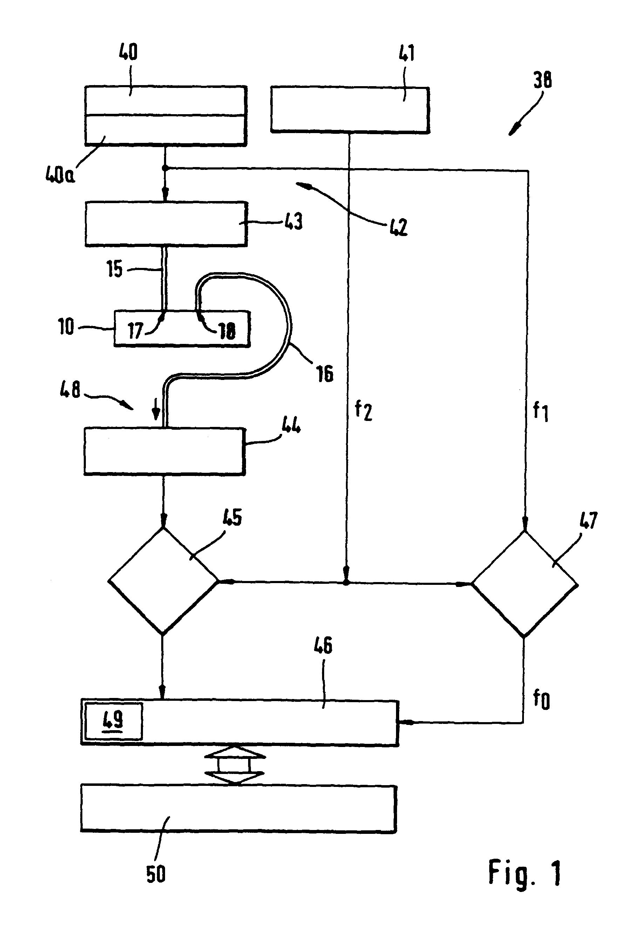

Figur 1 zeigt beispielhaft als Blockschaltbild den Aufbau einer Meßeinrichtung 38 zur FD-spektroskopischen Messung der Phasenverschiebung P und der Intensitätsmeßgrößen AC und DC. Das Meßprinzip der dargestellten Ausführungsform (Heterodyn-Verfahren) wird auch bei der Phasenfluorometrie angewendet.

Ein Frequenzgenerator 40 erzeugt eine hochfrequente periodische Schwingung, mit der über eine Verstärkerschaltung (Treiberstufe) 40a ein Lichtsender 43, beispielsweise eine Leuchtdiode, angesteuert wird. Dadurch wird das von dem Lichtsender 43 ausgehende Primärlicht mit einer hochfrequenten Frequenz f₁ moduliert, die als Trägerfrequenz bezeichnet wird. Die Frequenz sollte mehr als 50 MHz, vorzugsweise mehr als 200 MHz betragen. Das Licht des Lichtsenders 43 wird über ein erstes Glasfaser-Lichtleitkabel 15 an einem Einstrahlungsort 17 als Primärlicht in die biologische Matrix 10 eingestrahlt. Die durch die Bauteile 40, 40a, 43 und 15 gebildeten Lichteinstrahlungsmittel 42 können auch in anderer Weise realisiert sein. Diverse Möglichkeiten sind aus zahlreichen Publikationen bekannt.

Das an einem Detektionsort 18 aus der biologischen Probe 10 austretende Licht wird mit Detektionsmitteln 48 detektiert, die bei der dargestellten Ausführungsform ein Detektionslichtleitkabel 16 einschließen, durch das das Sekundärlicht an einen Lichtempfänger (Detektor) 44 übertragen wird. Der Lichtempfänger erzeugt ein der Lichtintensität entsprechendes elektrisches Ausgangssignal, das einem Signalmischer 45 zugeführt wird. An dem Signalmischer 45 liegt gleichzeitig das Ausgangssignal eines zweiten Frequenzgenerators 41 an, der mit dem Frequenzgenerator 40 synchronisiert ist und dessen Ausgangsfrequenz f₂ sich nur geringfügig (beispielsweise um f₀=1 kHz) von der Frequenz f₁ des ersten Frequenzgenerators 40 unterscheidet. Die Differenz-Frequenz f₀ ist so gewählt, daß sie einen Lock-in-Verstärker problemlos synchronisiert und das Eigenrauschen der erforderlichen Vorverstärker gering ist.

Das Ausgangssignal des Mischer 45 liegt am Eingang eines Lock-in-Verstärkers 46 an. Als Referenzsignal erhält der Lock-in-Verstärker 46 das unmittelbare Mischsignal der Frequenzgeneratoren 40 und 41, deren Ausgangssignale über einen zweiten Mischer 47 gemischt werden.

Der Lock-in-Verstärker 46 erzeugt zwei Ausgangssignale, die dem Realteil bzw. dem Imaginärteil des periodischen Meßsignals entsprechen. Daraus lassen sich die Phasendifferenz, die AC-Amplitude und die DC-Amplitude, jeweils bezogen auf das Referenzsignal, berechnen. Die hierzu erforderliche Recheneinheit 49 ist in der Figur symbolisch als Bestandteil des Lock-in-Verstärkers dargestellt. In der Praxis werden alle mathematischen Operationen von einem Mikrocomputer des Systems durchgeführt.

Die Meßeinrichtung kann auch in anderer Weise realisiert sein. Zahlreiche Varianten elektronischer Schaltungen zur Messung der Phasenverschiebung und der Intensitätsmeßgrößen AC und DC sind bekannt und können auch bei der vorliegenden Erfindung eingesetzt werden. Insofern wird insbesondere auf Publikationen zur Phasenfluorometrie und zur Frequenzdomänen-Spektroskopie (darunter die oben zitierten Artikel von Lakowicz und Duncan) Bezug genommen.

Zur Durchführung des zur Berechnung der Konzentration erforderlichen Auswerteschrittes (d.h. zur Ausführung eines Auswertealgorithmus) sind Auswertemittel 50, zweckmäßigerweise in Form des erwähnten Mikrocomputers, vorgesehen. Allgemein werden als Auswertemittel diejenigen Bestandteile eines Analysegerätes bezeichnet, die dazu dienen, aus gemessenen Werten der Meßgrößen die gesuchten Analysewerte zu ermitteln.

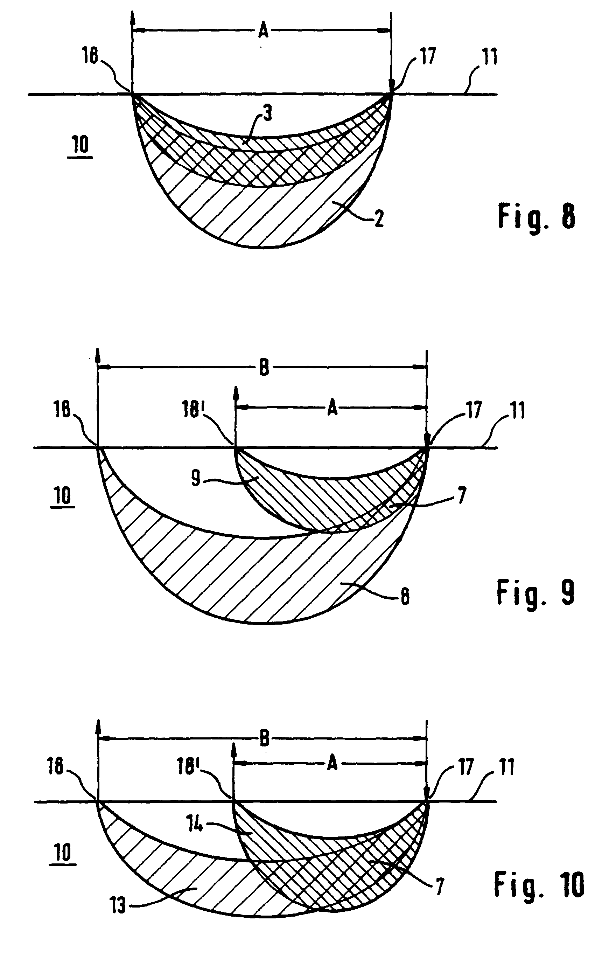

Bei der in Figur 1 dargestellten Ausführungsform ist nur ein Meßabstand zwischen dem Einstrahlungsort 17 und dem Detektionsort 18 vorgesehen. Dabei erfolgt die Einstellung unterschiedlicher Meßlichtwege innerhalb der biologischen Probe 10 durch Variation der Modulationsfrequenz (Trägerfrequenz), wie später anhand der Figuren 9 bis 11 noch näher erläutert wird.

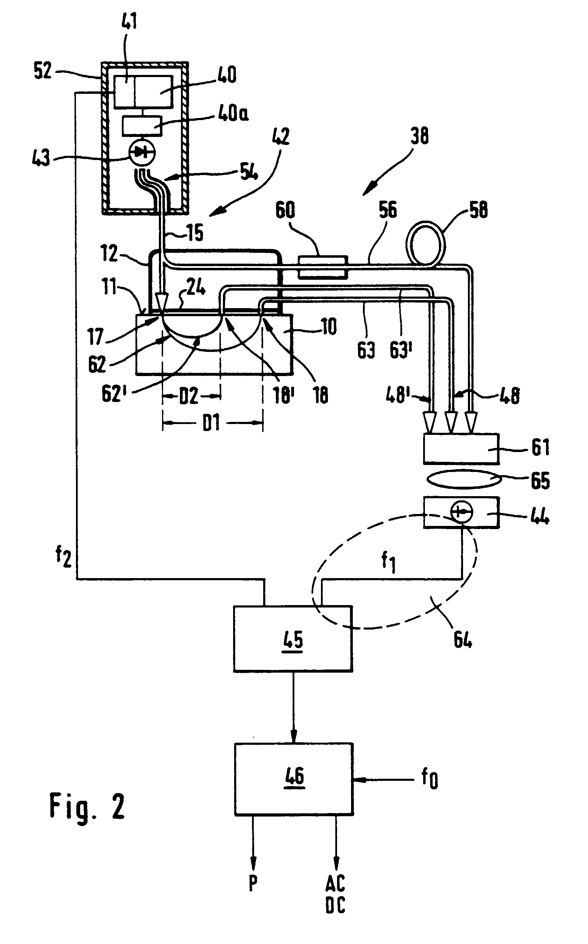

Figur 2 zeigt eine Ausführungsform, bei der an unterschiedlichen Detektionsorten austretendes Licht mit mehreren Detektionsmitteln erfaßt wird, wobei die Detektionsorte unterschiedliche Meßabstände zu einem gemeinsamen Einstrahlungsort haben. Daneben werden anhand von Figur 2 eine Reihe bevorzugter meßtechnischer Maßnahmen erörtert, die einzeln oder in Kombination miteinander verwendet werden können, um besonderen Bedingungen gerecht zu werden, die bei der Realisierung der Erfindung zu beachten sind. Wie erläutert sind die bekannten meßtechnischen Verfahren zur Bestimmung der Phasendifferenz sowie der Intensitätsmeßgrößen AC und DC prinzipiell auch im Rahmen der Erfindung verwendbar. Aus dem Anwendungsfall der Analyse biologischer Proben ergeben sich jedoch unter anderem die folgenden Bedingungen, die die Realisierung eines entsprechenden Systems erschweren.

Einerseits sind die Anforderungen an die Meßgenauigkeit sehr hoch. Beispielsweise muß die Phasenverschiebung zwischen dem eingestrahlten Primärlicht und dem detektierten Sekundärlicht mit einer Genauigkeit von typischerweise 0,1° bei einer Frequenz in der Größenordnung von 100 MHz gemessen werden. Bei kontinuierlicher Beobachtung muß der Phasenwinkel mit Integrationszeiten von einigen Minuten driftfrei über die gesamte Meßzeit gleichbleiben.

Als Lichtsender werden vorzugsweise Halbleiterlichtsender wie Leuchtdioden oder Laserdioden eingesetzt. Aus der damit erzielbaren geringen Intensität des Primärlichts resultiert eine sehr geringe Empfangslichtleistung an dem Lichtempfänger in der Größenordnung von nW (10⁻⁹ Watt).

Das Meßgerät soll möglichst klein, kompakt und kostengünstig sein. Die meßtechnischen Anforderungen sollen deswegen möglichst ohne teure Spezialbauteile erfüllt werden.

Eine erste Besonderheit der in Figur 2 dargestellten Meßeinrichtung 38 besteht darin, daß die beiden Frequenzgeneratoren 40, 41, die Treiberstufe 40a und die Primärlichtquelle 43 (bevorzugt eine Laserdiode) in einem elektromagnetisch isolierenden Gehäuse 52 angeordnet sind. Die elektromagnetische Isolierung des Gehäuses 52 erstreckt sich auch auf die Herausführung des Primärlichtes. Der Lichtleiter 15 wird durch einen für die elektromagnetische Hochfrequenzstrahlung praktisch undurchlässigen Kanal (Hochfrequenzfalle, RF-trap) 54 aus dem Gehäuse 52 herausgeführt. Damit wird erreicht, daß die im Inneren des Gehäuses 52 erzeugte Hochfrequenzleistung (die in der Größenordnung von 10 mW liegt) die hochempfindliche Phasenmessung nicht stört. Die Unterbringung der genannten Bauteile in einem elektromagnetisch isolierenden Gehäuse (beispielsweise einem vollständig geschlossenen Metallkasten) ist in diesem Sinne besonders vorteilhaft. Zumindest sollte die Treiberstufe 40a und die Primärlichtquelle 43, vorzugsweise auch der diese ansteuernde Frequenzgenerator 40 in gleicher Weise elektromagnetisch abgeschirmt sein.

Bei der dargestellten Ausführungsform befindet sich das Gehäuse 52 außerhalb des an der Grenzfläche 11 der Probe 10 mit einer Kontaktplatte 24 anliegenden Meßkopfes 12. Es kann jedoch auch darin integriert sein.

Eine zweite Besonderheit der in Figur 2 dargestellten Phasenmeßeinrichtung 38 ist darin zu sehen, daß ein Referenzlichtweg 56 vorgesehen ist, durch den das von der Primärlichtquelle 43 erzeugte Licht alternativ zu einem Probenlichtweg 63, der den durch die biologische Matrix 10 führenden Meßlichtweg 62 einschließt, zu dem gleichen Lichtempfänger 44 gelangen kann. Der Referenzlichtweg 56 weist in der dargestellten Ausführungsform eine optische Verzögerungsstrecke 58 (beispielsweise realisiert als Lichtleiter aus Lichtleitfasern) und einen optischen Abschwächer 60 auf. Diese Elemente sind so dimensioniert, daß der Referenzlichtweg 56 an den Probenlichtweg 63 angepaßt ist, wobei die Schwächung und Verzögerung des Lichts auf dem Probenlichtweg im wesentlichen durch den Meßlichtweg 62 innerhalb der biologischen Matrix bestimmt ist. Die Anpassung bedeutet, daß sowohl die Intensität als auch die Laufzeit des von der Primärlichtquelle 43 ausgehenden und auf einem der Lichtwege 56, 63 zu dem Lichtempfänger 44 gelangenden Lichts möglichst ähnlich ist. Vorzugsweise wird die Abschwächung und Verzögerung in dem Referenzlichtweg 56 auf die in der Praxis bei dem gegebenen Meßlichtweg 62 unter Meßbedingungen vorkommenden mittleren Werte eingestellt. Hinsichtlich der Lichtintensität sollte die entsprechende Abweichung höchstens +/- 50 % betragen. Die Verzögerung auf dem Referenzlichtweg 56 sollte an die Verzögerung auf dem Probenlichtweg 62 soweit angeglichen sein, daß die Phasenverschiebung auf beiden Wegen sich um maximal +/- 15°, bevorzugt +/- 5°, unterscheidet. Nach vorliegenden experimentellen Ergebnissen erfordert die Anpassung der Lichtwege eine Verzögerungsstrecke 58, die etwa viermal so groß wie der geometrische Abstand (Meßabstand) von Einstrahlungsort 17 und Detektionsort 18 ist. Die Verzögerungsstrecke ist dabei definiert durch die Differenz der Länge des Referenzlichtweges 56 im Vergleich zu dem außerhalb der Probe verlaufenden Teil des Probenlichtweges 63 (d.h. Probenlichtweg 63 abzüglich Meßlichtweg 62).

Der Referenzlichtweg 56 kann mit verhältnismäßig einfachen Mitteln hochstabil aufgebaut werden. Dadurch kann hinsichtlich der durch Änderungen der Analytkonzentration verursachten Änderungen der Phase mit relativ einfachen und damit kostengünstigen Mitteln eine erhöhte Meßgenauigkeit erreicht werden. Wichtig ist dabei, daß der Probenlichtweg 63 und der Referenzlichtweg 56 auf optischem Wege auf den gleichen Lichtempfänger 44 umschaltbar sind. Dies ist in der dargestellten Ausführungsform mit Hilfe eines LCD-optischen Schalters 61 und einer Linse 65 realisiert.

Der LCD-optische Schalter 61 dient bei der dargestellten Ausführungsform auch zur Einstellung von zwei unterschiedlichen Meßlichtwegen 62 und 62' in der biologischen Probe 10. Ein im Vergleich zu dem Meßabstand D1 kleinerer Meßabstand D2 mit einem entsprechenden Meßlichtweg 62' wird dadurch eingestellt, daß das an dem Detektionsort 18' austretende Licht auf einem weiteren Probenlichtweg 63' (zweckmäßigerweise mit Hilfe einer Lichtleitfaser) dem gleichen LCD-optischen Umschalter 61 zugeführt wird. Hier bilden also die Lichtleitfasern der Probenlichtwege 63 und 63' in Verbindung mit für beide Probenlichtwege gemeinsamen Elementen (optischer Schalter 61, Linse 65 und Lichtempfänger 44) zwei unterschiedliche Detektionsmittel 48,48' zur Erfassung von Sekundärlicht, das an den beiden unterschiedlichen Detektionsorten 18 und 18' aus der biologischen Matrix austritt. Dabei kann die gleiche Phasenmeßvorrichtung 38 verwendet werden, um die Meßgrößen Phasendifferenz (P), AC-Amplitude (AC) und DC-Amplitude (DC) für unterschiedliche Meßabstände und (bei Realisierung der hier diskutierten bevorzugten Ausführungsform) einen optischen Referenzlichtweg 56 zu bestimmen. Es kann vorteilhaft sein, in dem Probenlichtweg 63', der das intensitätsstärkere Signal transportiert, einen nicht dargestellten optischen Abschwächer und/oder eine ebenfalls nicht dargestellte Verzögerungsstrecke vorzusehen, um Intensität und Phase der an dem Lichtempfänger 44 ankommenden Signale aneinander und - bei Realisierung der dargestellten Ausführungsform mit einem zusätzlichen Referenzlichtweg - an das Referenzsignal anzupassen.

Eine dritte Besonderheit der in Figur 2 dargestellten Ausführungsform besteht darin, daß der Signalweg 64 zwischen dem Lichtempfänger 44 und dem Signalmischer 45 sehr kurz ist. Im Rahmen der vorliegenden Erfindung wurde erkannt, daß die Empfindlichkeit der Lichtdetektion durch diese Maßnahme positiv beeinflußt wird. Ein möglichst kurzer Signalweg auf dem Abschnitt zwischen den Bauelementen 44 und 45 führt zu einer geringen Leitungskapazität. Dadurch ist es möglich, trotz der erforderlichen hohen Frequenzen mit einer verhältnismäßig hohen Impedanz des Mischers beziehungsweise eines diesem vorgeschalteten Signalvorverstärkers zu arbeiten, was wiederum hinsichtlich der Signalstärke bei gegebenem Strom der Photodiode vorteilhaft ist. Die nutzbare Empfindlichkeit des an sich einfachen und damit kostengünstigen Halbleiter-Photoempfängers kann somit durch eine einfache Maßnahme erheblich erhöht werden.

Vorzugsweise beträgt der Signalweg zwischen Lichtempfänger 44 und Mischer 45 weniger als 1 cm. Besonders bevorzugt ist der Lichtempfänger 44 und der Signalmischer 45 gemeinsam auf dem gleichen Halbleitersubstrat integriert. Dabei sind mehrere unterschiedliche schaltungstechnische Realisierungen vorteilhaft. Es kann ein Verstärker mit nachgeschaltetem Mischer (z.B. einem "balanced mixer") eingesetzt werden. Es kann ein FET-Mischer verwendet werden, an dessen Gate der Lichtempfänger 44 direkt angekoppelt ist. Schließlich kann eine als Photoempfänger verwendete Photodiode selbst Bestandteil des Mischers 45 sein. Beispielsweise kann eine der Dioden eines balanced mixer durch die Photodiode ersetzt werden.

Der Lichtempfänger 44 ist besonders bevorzugt eine Avalanche-Photodiode. Eine insbesondere in Verbindung mit einer Avalanche-Photodiode bevorzugte Möglichkeit besteht darin, die Empfindlichkeit des Lichtempfängers 44 unmittelbar mit der Frequenz f₂ zu modulieren. Dies kann im Falle eines Photomultipliers dadurch geschehen, daß f₂ an dessen Dynode angelegt wird. Im Falle eines Halbleiter-Lichtempfängers kann dessen Versorgungsspannung mit f₂ moduliert werden. Dadurch werden die Funktionen des Lichtempfängers 44 und des Mischers 45 in einem Bauteil kombiniert.

Die geometrischen Bedingungen hinsichtlich der Abmessungen des Einstrahlungsortes 17 und des Detektionsortes 18, hinsichtlich der Anordnung dieser Orte auf der Grenzfläche 11 der biologischen Matrix 10 und insbesondere hinsichtlich des Meßabstandes D können im Einzelfall empirisch festgelegt werden. Dabei sind folgende Grundregeln zu beachten.

Die Begriffe "Einstrahlungsort" und "Detektionsort" sind geometrisch zu verstehen, nämlich als der Teilbereich der Grenzfläche einer biologischen Matrix, an dem Lichtstrahlen, die bei dem jeweiligen Detektionsschritt für den ermittelten Laufzeit-Parameter bestimmend sind, durch die Grenzfläche hindurchtreten. Soweit nachfolgend Angaben über Distanzen zwischen Einstrahlungsort und Detektionsort gemacht werden, beziehen sich diese auf die Mitte des jeweiligen Einstrahlungsortes bzw. Detektionsortes, jeweils betrachtet in Richtung des Abstandes (d.h. der kürzesten Verbindung) zwischen Einstrahlungsort und Detektionsort.

Um die Meßgrößen mit guter Auflösung einem bestimmten Meßabstand zuordnen zu können, sollte die Ausdehnung des Einstrahlungsortes und des Detektionsortes in Richtung des genannten Abstandes nicht zu groß sein. Vorzugsweise beträgt sie weniger als 2 mm, besonders bevorzugt weniger als 1 mm.

Die Meßlichtwege der innerhalb eines Detektionsschrittes durchgeführten Messungen, deren Ergebnisse zur Ermittlung eines Absorptionsparameters und/oder Streuparameters verwendet werden, sollten sich hinreichend deutlich unterscheiden. Allgemein kann man sagen, daß der Unterschied groß genug sein muß, daß der Meßfehler bei der Bestimmung der Einzelmeßwerte (für unterschiedliche Meßlichtwege) deutlich kleiner ist, als die Differenz der Meßwerte. Vorzugsweise sollten die zur Berechnung eines Absorptionsparameters oder eines Streuparameters verwendeten Intensitätsmeßwerte sich um mindestens 10 % unterscheiden. Entsprechend groß sollte der Unterschied der Meßlichtwege sein.

Der größtmögliche Meßabstand wird durch die abnehmende Intensität des Sekundärlichts und die daraus resultierende Verschlechterung des Signal/Rauschverhältnisses bestimmt. Die Obergrenze läßt sich im Einzelfall empirisch bestimmen. Vorzugsweise sollte der Meßabstand kleiner als 4 cm sein. Nach den vorliegenden Erkenntnissen wird eine optimale Meßgenauigkeit bei Meßabständen um 2 cm herum (etwa zwischen 1 cm und 3 cm) erreicht.

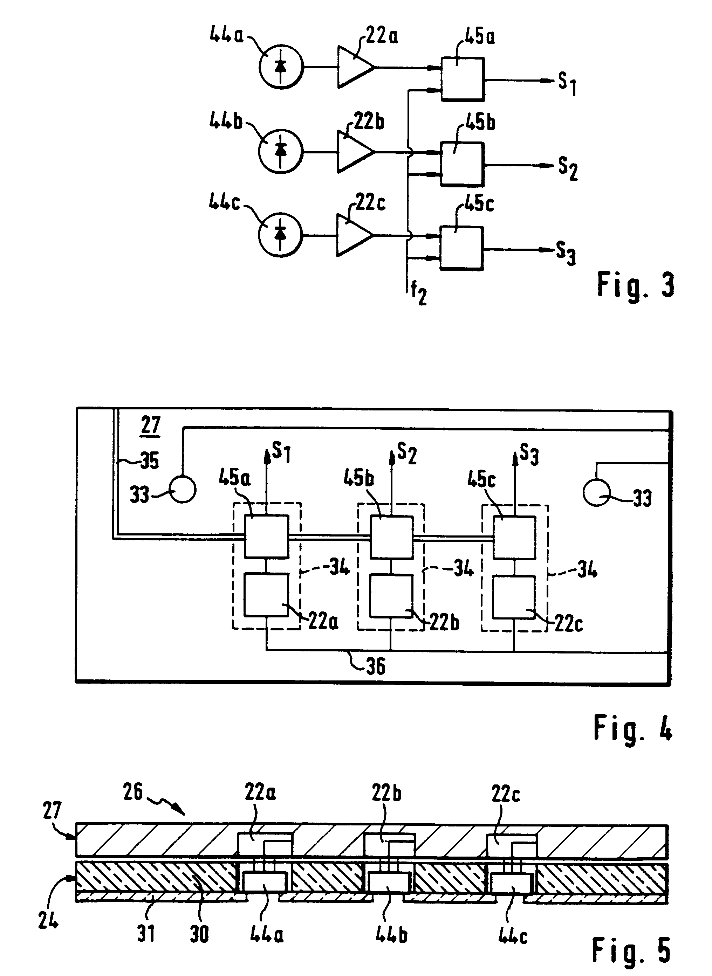

Die Figuren 3 bis 7 zeigen vorteilhafte Einzelheiten einer PDOLS-Meßvorrichtung.

Gemäß Figur 3 ist jedem Lichtempfänger 44a,44b,44c ein Hochfrequenz-Vorverstärker 22a,22b,22c nachgeschaltet. Das Ausgangssignal der Hochfrequenz-Verstärker 22a,22b,22c wird jeweils einem Signalmischer 45a,45b,45c zugeführt, an dessen jeweils anderem Eingang die Frequenz f₂ (einer Schaltung gemäß Figur 1) anliegt. Die Signalwege zwischen den Lichtempfängern und den Hochfrequenz-Vorverstärkern sowie zwischen den Hochfrequenz-Vorverstärkern und den Signalmischern sollten aus den bereits diskutierten Gründen möglichst kurz sein. Bevorzugt beträgt die Gesamtlänge des Signalweges von dem Lichtempfänger bis zu dem Mischer weniger als 1 cm, besonders bevorzugt weniger als 3 mm. Die Ausgangssignale S₁,S₂,S₃ der Mischer 45a,45b,45c sind niederfrequent und können deswegen mit den üblichen Mitteln der Niederfrequenzschaltungstechnik weiterbearbeitet werden.

In den Figuren 4 und 5 ist die Kontaktplatte 24 dargestellt, welche zur Durchführung einer Analyse gegen die Grenzfläche 11 der biologischen Probe 10 gedrückt wird. Die Kontaktplatte 24 gehört zu einem für sämtliche Lichtempfänger 44a,44b und 44c gemeinsamen Trägerbauteil 26. Das Trägerbauteil 26 umfaßt außer der Kontaktplatte 24 eine Elektronik-Platine 27, die in Multilayer-Technik ausgeführt ist. Sie kann als gedruckte Schaltung (printed circuit board) oder in Hybrid-Technik ausgeführt sein. Die Kontaktplatte 24 ist ihrerseits mehrschichtig aufgebaut und besteht aus einer Platte aus schwarz eingefärbtem Glas ("Schwarzglasplatte") 30 und einer Metallplatte 31. In passenden Ausnehmungen der Schwarzglasplatte 30 sind Avalanche-Photodioden als Lichtempfänger 44 angeordnet, deren lichtempfindliche Fläche der Probe zugewandt ist.

Die Elektronik-Platine 27 des Trägerbauteils 26 dient als Träger für die HF-Vorverstärker 22a,22b,22c und die Signalmischer 45a,45b,45c, wobei jeweils der einem Lichtempfänger 44 zugeordnete Vorverstärker 22 und Mischer 45 sich in einem Metall-Abschirmgehäuse 34 befinden. Die Zuführung der Hochfrequenz f₂ zu den Mischern 45a,45b,45c erfolgt über eine als Streifenleiter ausgeführte 50 Ohm-Leitung, die in der Elektronik-Platine 27 verläuft. Die Versorgungsspannung für die Avalanche-Photodioden wird über eine ebenfalls in der Elektronikplatine 27 verlaufende Leitung 36 zugeführt. Auf der Elektronik-Platine ist zweckmäßigerweise auch die weitere Schaltung zur Verarbeitung der Niederfrequenzsignale untergebracht, die jedoch der Übersichtlichkeit halber nicht dargestellt ist.

An die Elektronikplatine 27 des Trägerbauteils 26 sind zwei Temperaturmeßelemente 33 thermisch angekoppelt. Sie dienen dazu, die Temperatur des Trägerbauteiles 26 zu kontrollieren. Dadurch können durch Temperaturschwankungen verursachte Änderungen der Empfindlichkeit der Lichtempfänger 44 bei der Bestimmung des Absorptions- bzw. Streuparameters und allgemein in dem Auswerteschritt berücksichtigt werden.

Durch die gemeinsame Integration der Lichtempfänger 44a,44b,44c in einem einzigen Bauteil, vorzugsweise zusammen mit den erforderlichen Vorverstärkern und den Mischern wird gewährleistet, daß alle Avalanche-Photodioden die gleiche relative Phasenlage haben und die Verarbeitungskanäle der Detektionsmittel für die unterschiedlichen Detektionsorte eine weitestgehend gleiche Charakteristik aufweisen. Daneben ergibt sich eine kompakte Bauweise. Die Multilayer-Technik ermöglicht es, die Hochfrequenzsignale abgeschirmt zuzuführen und erlaubt eine sehr kurze Verbindung zwischen den Lichtempfängern und den Mischern. Sofern FET-Mischer verwendet werden, kann der gesonderte Vorverstärker entfallen. In diesem Fall soll der Abstand zwischen dem Lichtempfänger und dem jeweils zugeordneten Mischer minimiert sein.

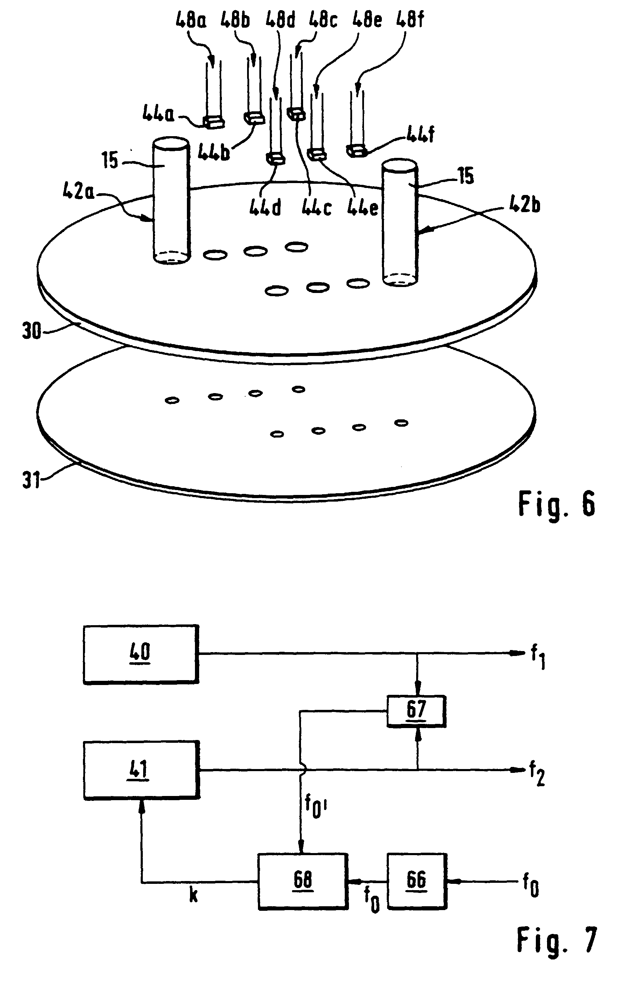

Figur 6 zeigt in Form einer Explosionszeichnung die Anordnung von zwei Einstrahlungsmitteln 42a,42b und sechs Detektionsmitteln 48a bis 48f in der Kontaktplatte einer Meßeinrichtung.

Die Kontaktplatte besteht ebenso wie bei der Ausführungsform der Figur 5 aus einer Schwarzglasplatte 30 und einer Metallplatte 31. In der Schwarzglasplatte befinden sich Ausnehmungen, die auf die Größe der darin eingesetzten Elemente angepaßt sind. In der Figur sind von den Einstrahlungsmitteln 42a,42b Sender-Lichtleitkabel 15 dargestellt, über die Licht von einem in der Figur nicht dargestellten Lichtsender an zwei verschiedenen Einstrahlungsorten 17a,17b eingestrahlt werden kann. Die Größe des Einstrahlungsortes wird durch den Durchmesser der entsprechenden Öffnungen in der Metallplatte 31 bestimmt und beträgt vorzugsweise weniger als 1 mm, besonders bevorzugt etwa 0,5 mm.

Die Lichtempfänger 44a bis 44f sind bei der dargestellten Ausführungsform ohne zwischengeschaltete Lichtleitfasern unmittelbar an der mit der Probe in Kontakt stehenden Fläche der Kontaktplatte 24 angeordnet. Sie sitzen in entsprechenden Ausnehmungen der Schwarzglasplatte 30, wobei die Größe der entsprechenden Detektionsorte 18a bis 18f wiederum durch die Größe der Öffnungen in der Maske 32 bestimmt wird. Sie liegt vorzugsweise unter 2 mm, besonders bevorzugt bei etwa 1 mm. Die Verwendung von mehreren Einstrahlungsmitteln und mehreren Detektionsmitteln in einer Anordnung, bei der der gleiche Meßabstand durch mehrere unterschiedliche Kombinationen von Einstrahlungsorten und Detektionsorten eingestellt werden kann, ermöglicht eine meßtechnische Redundanz, wie sie in der bereits zitierten WO 94/10901 erläutert wird.

Die Genauigkeit der Phasenmessung mit einer Phasenmeßeinrichtung, die nach dem Heterodyn-Prinzip arbeitet, ist wesentlich davon abhängig, daß die Differenzfrequenz fₒ zwischen den beiden Frequenzgeneratoren (f₁, f₂) 40 und 41 ("Kreuzkorrelationsfrequenz") über die Meßzeit konstant bleibt. Da andererseits fₒ (beispielsweise 1 KHz) nur einen kleinen Bruchteil der Absolutfrequenzen der Frequenzgeneratoren 40, 41 (beispielsweise 100,00 MHz und 100,001 MHz) ausmacht, müßten in den Frequenzgeneratoren 40, 41 hochstabile und deswegen aufwendige Oszillatoren eingesetzt werden.

Gemäß einer bevorzugten Ausführungsform der Erfindung wird statt dessen die Differenzfrequenz f₀ von einem Differenzfrequenzgenerator 66 vorgegeben und die Frequenz des zweiten Meßfrequenzgenerators f₂ auf Basis der vorgegebenen Differenzfrequenz so geregelt, daß der zweite Frequenzgenerator 41 Frequenzschwankungen des ersten Frequenzgenerators 40 mit konstanter Differenzfrequenz folgt.

Bei der Ausführungsform gemäß Figur 7 ist dies dadurch realisiert, daß die Frequenzen f₁ und f₂ der Generatoren 40, 41 an den Eingängen eines Signalmischers 67 anliegen, an dessen Ausgang die jeweils aktuelle Differenzfrequenz f₀, erzeugt wird. Diese wird als Ist-Signal einer Regeleinheit 68 zugeführt, welcher andererseits das Signal des Differenzfrequenzgenerators 66 als Sollwert zugeführt wird. Die Regeleinheit 68 vergleicht das Ist-Signal f₀, mit dem Sollsignal f₀ und erzeugt ein Korrektursignal k, welches einem der beiden Frequenzgeneratoren (in der Figur dem Frequenzgenerator 41) zugeführt wird, um dessen Frequenz f₂ so zu regeln, daß die Differenz zwischen f₁ und f₂ exakt der vorgegebenen Differenzfrequenz f₀ entspricht. Die Regelung erfolgt bevorzugt in bekannter Weise mit dem PLL-Prinzip.

Anhand der Figuren 8 bis 11 werden Möglichkeiten zur bewegungslosen Beeinflussung des Lichtweges innerhalb einer biologischen Probe 10 erläutert.

Figur 8 zeigt in einer Schnittdarstellung den optischen Lichtweg in einer biologischen Probe 10 für zwei unterschiedliche Modulationsfrequenzen f₁ und f₂, wobei der Lichtweg mit der niedrigeren Modulationsfrequenz f₁ als 2 und der Lichtweg mit der höheren Modulationsfrequenz f₂ als 3 bezeichnet ist. Das Licht wird dabei jeweils an dem gleichen Einstrahlungsort 17 der Oberfläche 11 mit einem nicht dargestellten Lichteinstrahlungsmittel eingestrahlt und an dem gleichen Detektionsort 18 der Oberfläche 11 gemessen, so daß der Meßabstand A in beiden Fällen gleich ist.

Der "effektive optische Weg" in dem Gewebe kann beispielsweise als das Teilvolumen der Probe definiert werden, in dem ein bestimmter Prozentsatz (z.B. 90 %) des Lichts verläuft, das von dem Einstrahlungsort 17 zu dem Detektionsort 18 gelangt und zu dem von dem Lichtempfänger erzeugten elektrischen Signal beiträgt. Aufgrund der durch die Vielfachstreuung in der Probe bedingten geometrischen Verhältnisse hat dieses Volumen eine etwa bananenförmige Gestalt, wenn die Einstrahlungsstelle und die Lichtquelle (wie dargestellt) auf der gleichen Oberfläche 11 der Gewebe-Probe 10 liegen.

Dabei ist die Form des effektiven optischen Lichtweges eine komplexe Funktion des Meßabstandes zwischen dem Einstrahlungsort 17 und dem Detektionsort 18, der Absorptions- und Streueigenschaften der Probe (einschließlich deren möglicher lokaler Unterschiede) und der Modulationsfrequenz. Man kann jedoch als allgemeine Regel sagen, daß die maximale Tiefe des optischen Weges 2,3 in dem Gewebe mit zunehmendem Abstand A und mit abnehmender Frequenz f größer wird, während sie umgekehrt kleiner wird, wenn man einen kleineren Meßabstand und eine höhere Frequenz verwendet. Auf Basis dieser Information ist es möglich, durch Abstimmung des Meßabstandes A und der Modulationsfrequenz f den Lichtweg in der Probe so zu bestimmen, daß er im wesentlichen in einem vorbestimmten (gewünschten) Teilvolumen des Gewebes verläuft.

Figur 9 zeigt zwei Lichtwege 8,9 für gleiche Modulationsfrequenzen, jedoch unterschiedliche Meßabstände A bzw. B, wobei im dargestellten Fall das Licht an einem Einstrahlungsort 17 eingestrahlt und an zwei unterschiedlichen Detektionsorten 18,18' detektiert wird. Man erkennt, daß entsprechend der vorstehend erläuterten allgemeinen Regel die maximale Tiefe des Lichtweges bei dem größeren Meßabstand B größer ist. Die beiden Lichtwege überlappen sich nur in einem verhältnismäßig kleinen Teilvolumen 7. Dies ist für viele Anwendungszwecke nachteilig, weil die jeweils gemessenen Meßgrößen sich auf verschiedene Meßvolumina beziehen.

In diesem Sinne vorteilhafter ist die in Figur 10 dargestellte Möglichkeit, bei unterschiedlichen Meßabständen A und B zugleich für den größeren Meßabstand (Lichtweg 13) eine höhere Modulationsfrequenz als für den kürzeren Meßabstand B (Lichtweg 14) zu verwenden. Dadurch wird ein erheblich größeres Überlappungsvolumen 7 erreicht.

Durch geeignete Wahl des Meßabstandes und der Modulationsfrequenz ist es somit möglich, insbesondere in Schichtstrukturen, wie sie die Haut darstellt, die effektiven Lichtwege so einzustellen, daß hauptsächlich eine bestimmte Schicht meßtechnisch erfaßt wird. Nach neueren Erkenntnissen kann es vorteilhaft sein, die Messung nicht primär auf das Hautgewebe, sondern auf das darunterliegende Fett- oder Muskelgewebe zu konzentrieren.

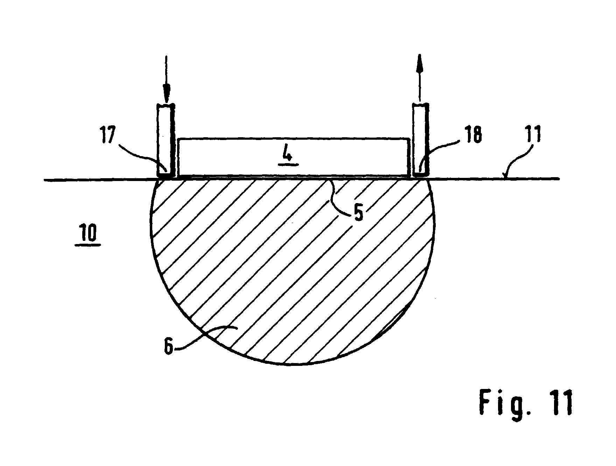

Eine weitere Möglichkeit zur Beeinflussung des effektiven Lichtweges ist in Figur 11 stark schematisiert dargestellt. Dabei erstreckt sich zwischen dem Einstrahlungsort 17 und dem Detektionsort 18 ein Bauelement 4 mit einer auf der Gewebeoberfläche 11 aufliegenden reflektierenden Oberfläche 5. Technisch kann dies z.B. durch eine reflektierende Beschichtung der auf die Gewebeoberfläche 11 aufgelegten Kontaktplatte des Sensor-Meßkopfes realisiert sein. Durch die reflektierende Oberfläche 5 werden an die Oberfläche 11 der Probe 10 gelangende Lichtanteile in das Gewebe zurückreflektiert und tragen somit zu dem an dem Detektionsort 18 detektierten Signal bei, so daß die dargestellte Form des effektiven optischen Lichtweges 6 resultiert, die das Volumen bis unmittelbar an die Oberfläche 11 einschließt. Auch diese Maßnahme kann vorteilhaft verwendet werden, um den Lichtweg in dem Gewebe auf ein gewünschtes vorbestimmtes Teilvolumen einzustellen.

Es wird deutlich, daß durch Variation der Modulationsfrequenz bei gegebener Position des Einstrahlungsortes und des Detektionsortes (und unveränderlichen Meßabstand) unterschiedlicher Meßlichtwege in der biologischen Probe 10 eingestellt werden können. Darüber hinaus ist es möglich, das Teilvolumen, in dem der effektive Meßlichtweg verläuft, zu wählen. Die Wahl des Teilvolumens in dem Gewebe kann bei in vivo-Analysen von erheblicher Bedeutung für die Meßgenauigkeit sein. Insbesondere sollte angestrebt werden, die Messung auf ein möglichst präzise vorbestimmtes Teilvolumen der Haut zu beschränken, um unterschiedliche Einflüsse durch die Heterogenität unterschiedlicher Gewebeschichten zu reduzieren. Eine Konzentration der Messung auf das Kapillarbett der Haut ist besonders bevorzugt.

## Patentansprüche

1. Verfahren zur analytischen Bestimmung der Konzentration eines Analyten in einer biologischen Probe, bei welchem
in einem Detektionsschritt Licht mittels Einstrahlungsmitteln durch eine die biologische Probe begrenzende Grenzfläche als Primärlicht an einem Einstrahlungsort in die Probe eingestrahlt und aus der biologischen Probe durch eine diese begrenzende Grenzfläche an einem Detektionsort in einem vorbestimmten Meßabstand von dem Einstrahlungsort austretendes Licht mittels Detektionsmitteln als Sekundärlicht detektiert wird, um eine durch die Wechselwirkung mit der biologischen Probe veränderliche, meßbare physikalische Eigenschaft des Lichtes als Meßgröße zu bestimmen, die mit der Konzentration des Analyten in der biologischen Probe korreliert und
in einem Auswerteschritt die Analytkonzentration auf Basis der in dem Detektionsschritt gemessenen Meßgröße ermittelt wird,
**dadurch gekennzeichnet**, daß in dem Detektionsschritt
(a) ohne Bewegung der Lichteinstrahlungsmittel oder der Detektionsmittel Frequenzdomänen-spektroskopische Messungen mit mindestens zwei unterschiedlichen Meßlichtwegen zwischen Einstrahlungsort und Detektionsort durchgeführt werden,
(b) jede der Frequenzdomänen-spektroskopischen Messungen bei mindestens zwei unterschiedlichen Lichtwellenlängen durchgeführt wird, und
(c) bei den Frequenzdomänen-spektroskopischen Messungen jeweils die Phasenverschiebung des Sekundärlichts gegenüber dem Primärlicht sowie eine Intensitätsmeßgröße bestimmt wird.

2. Verfahren nach Anspruch 1, **dadurch gekennzeichnet**, daß in dem Auswerteschritt aus den Meßwerten der Frequenzdomänen-spektroskopischen Messungen ein Absorptionsparameter, der ein Maß der Absorption des Lichts in der biologischen Probe ist und/oder ein Streuparameter, der ein Maß der Streuung des Lichts in der biologischen Probe ist, als von dem jeweils anderen Parameter unabhängige Meßgröße bestimmt wird, wobei aus mindestens einem der Parameter die Analytkonzentration abgeleitet wird.

3. Verfahren nach einem der vorhergehenden Ansprüche, **dadurch gekennzeichnet**, daß die Intensitätsmeßgröße die AC-Intensitätskomponente des Sekundärlichts ist.

4. Verfahren nach einem der vorhergehenden Ansprüche, **dadurch gekennzeichnet**, daß die Intensitätsmeßgröße die DC-Intensitätskomponente des Sekundärlichts ist.

5. Verfahren nach einem der vorhergehenden Ansprüche, **dadurch gekennzeichnet**, daß bei den Frequenzdomänen-spektroskopischen Messungen zur bewegungslosen Einstellung der unterschiedlichen Meßlichtwege an unterschiedlichen Detektionsorten austretendes Licht mit mehreren Detektionsmitteln erfaßt wird, wobei die Detektionsorte unterschiedliche Meßabstände zu mindestens einem Einstrahlungsort haben.

6. Verfahren nach einem der vorhergehenden Ansprüche, **dadurch gekennzeichnet**, daß bei den Frequenzdomänen-spektroskopischen Messungen zur bewegungslosen Einstellung unterschiedlicher Meßlichtwege bei gleichem Meßabstand zwischen Einstrahlungsort und Detektionsort das Primärlicht mit unterschiedlichen Modulationsfrequenzen moduliert wird.

7. Verfahren nach einem der vorhergehenden Ansprüche, **dadurch gekennzeichnet**, daß die Konzentration des Analyten im wesentlichen aus dem Absorptionsparameter abgeleitet wird, wobei der Analyt insbesondere Wasser, Glucose, Bilirubin, Cholesterin, Triglyceride, Harnstoff, Harnsäure, Oxihämoglobin, Hämoglobin oder Cytochrom-Oxidase ist.

8. Verfahren nach einem der vorhergehenden Ansprüche, **dadurch gekennzeichnet**, daß die biologische Probe eine biologische Flüssigkeit, insbesondere Blut, ist.

9. Verfahren nach einem der Ansprüche 1 bis 7, **dadurch gekennzeichnet**, daß die biologische Probe ein biologisches Gewebe, insbesondere der Fingerbeere, der Oberbauchdecke, des Nagelbettes, der Lippe, der Zunge des inneren Oberarms, der Muskeln, der Skleren oder subcutanes Fettgewebe ist.

10. Verfahren nach einem der vorhergehenden Ansprüche, **dadurch gekennzeichnet**, daß der biologischen Probe eine exogene Substanz zugeführt wird, die den Streuparameter und/oder den Absorptionsparameter in dem Gewebe beeinflußt und die Konzentration der exogenen Substanz in einem Teilvolumen des Gewebes als Analyt bestimmt wird.

11. Verfahren nach einem der vorhergehenden Ansprüche, **dadurch gekennzeichnet**, daß die Meßlichtwege in dem Gewebe auf ein vorbestimmtes Teilvolumen des Gewebes eingestellt werden, in dem die Analytkonzentration bestimmt werden soll.

12. Verfahren nach Anspruch 11, **dadurch gekennzeichnet**, daß das Teilvolumen das Kapillarbett der Haut ist.

13. Verfahren nach Anspruch 11 oder 12, **dadurch gekennzeichnet**, daß als Mittel zur Einstellung des Teilvolumens eine auf der Gewebeoberfläche aufliegende, reflektierende Fläche vorgesehen ist.

14. Verfahren nach einem der Ansprüche 6 bis 13, **dadurch gekennzeichnet**, daß die Modulationsfrequenz und der Meßabstand so aufeinander abgestimmt werden, daß der Lichtweg im wesentlichen in dem vorbestimmten Teilvolumen des Gewebes verläuft.

15. Vorrichtung zur Durchführung des Verfahrens nach einem der vorhergehenden Ansprüche, mit
einem zum Anlegen an eine Grenzfläche der biologischen Probe vorgesehenen Meßkopf (12),
einer Meßeinrichtung (38) zur Bestimmung einer durch die Wechselwirkung des Lichts mit der biologischen Probe (10) veränderlichen physikalischen Eigenschaft des Lichtes als Meßgröße, die mit der Konzentration des Analyten in der biologischen Probe (10) korreliert, mit
- Einstrahlungsmitteln (42) mit einem Lichtsender (43) zum Einstrahlen von Primärlicht in die biologische Probe (10) an einem Einstrahlungsort (17) durch eine die biologischen Probe (10) begrenzende Grenzfläche (11), und
- Detektionsmitteln (48) mit einem Lichtempfänger (44) zum Detektieren von aus der biologischen Probe (10) durch eine diese begrenzende Grenzfläche (11) an einem Detektionsort (18) austretendem Sekundärlicht, und
Auswertemitteln (50) zur Ermittlung der Analytkonzentration aus dem Meßwert der Meßgröße,
**dadurch gekennzeichnet**, daß
die Meßeinrichtung (38)
- einen Frequenzgenerator (40) zur Steuerung des Primärlichtsenders (3) einschließt, wodurch das Primärlicht mit einer hochfrequenten Trägerfrequenz moduliert ist,
- eine Phasenmeßeinrichtung und eine Intensitätsmeßeinrichtung einschließt, um die Phasenverschiebung des Sekundärlichts gegenüber dem Primärlicht und eine Intensitätsmeßgröße des Sekundärlichts als Meßgrößen zu bestimmen, und
- so ausgebildet ist, daß die Meßgrößen ohne Bewegung der Einstrahlungsmittel (42) und der Detektionsmittel (48) bei mindestens zwei unterschiedlichen Meßlichtwegen (62,62') zwischen Einstrahlungsort (17) und Detektionsort (18) bei jeweils mindestens zwei Lichtwellenlängen bestimmbar sind.

16. Vorrichtung nach Anspruch 15, **dadurch gekennzeichnet**, daß der Lichtsender (43) von dem Frequenzgenerator (40) über eine Treiberstufe (40a) angesteuert wird und die Treiberstufe (40a) und der Lichtsender (43), bevorzugt auch der Frequenzgenerator (40), in einem elektromagnetisch isolierenden Gehäuse angeordnet sind.

17. Vorrichtung nach einem der Ansprüche 15 bis 16, **dadurch gekennzeichnet**, daß die Meßeinrichtung (38) einen zweiten Frequenzgenerator (41) einschließt, der ebenfalls in einem elektromagnetisch isolierenden Gehäuse (52) angeordnet ist.

18. Vorrichtung nach einem der Ansprüche 15 bis 17, **dadurch gekennzeichnet**, daß die Meßeinrichtung eine Mehrzahl von Detektionsmitteln (48a,48b...) mit jeweils einem Lichtempfänger (44a,44b,...) aufweist, die an unterschiedlichen Detektionsorten aus der biologischen Probe austretendes Licht simultan detektieren und die Lichtempfänger (44a,44b,...) auf einem gemeinsamen Trägerbauteil (26) angeordnet sind.

19. Vorrichtung nach Anspruch 18, **dadurch gekennzeichnet**, daß an das Trägerbauteil (26) ein Temperaturmeßelement (33) thermisch angekoppelt ist, dessen Ausgangssignal den Auswertemitteln (50) zugeführt wird, um die Temperatur der Lichtempfänger (44) bei der Ermittlung der Analytkonzentration zu berücksichtigen.

20. Vorrichtung nach einem der Ansprüche 15 bis 19, **dadurch gekennzeichnet**, daß einem Lichtempfänger (44) in der Meßeinrichtung ein Hochfrequenzverstärker nachgeschaltet ist und der Abstand zwischen dem Lichtempfänger und dem nachgeschalteten Hochfrequenz-verstärker minimiert ist.

21. Vorrichtung nach einem der Ansprüche 15 bis 20, **dadurch gekennzeichnet**, daß das Ausgangssignal eines Lichtempfängers (44) in der Meßeinrichtung einem Signalmischer (45) zugeführt wird, und der Signalweg (64) zwischen dem Lichtempfänger (44) und dem Signalmischer (45) minimiert ist.

22. Vorrichtung nach Anspruch 21, **dadurch gekennzeichnet**, daß der Signalmischer (45) ein FET-Mischer ist und der Lichtempfänger (44) direkt an das Gate des FET-Mischers angekoppelt ist.

23. Vorrichtung nach Anspruch 21, **dadurch gekennzeichnet**, daß der Lichtempfänger (44) eine Photodiode ist und die Photodiode ein Bestandteil des Signalmischers (45) ist.

24. Vorrichtung nach einem der Ansprüche 15 bis 23, **dadurch gekennzeichnet**, daß der Meßkopf (12) mehrere Detektionsmittel (48,48') einschließt, die über mehrere Probenlichtwege (63,63') an unterschiedlichen Detektionsorten (18,18') aus der biologischen Probe (10) austretendes Licht detektieren, wobei das Licht zwischen den Probenlichtwegen (63,63') optisch auf einen gemeinsamen Lichtempfänger (44) umschaltbar ist.

25. Vorrichtung nach einem der Ansprüche 15 bis 24, **dadurch gekennzeichnet**, daß die Meßeinrichtung eine optische Referenzlichtstrecke (56) einschließt, die hinsichtlich der Lichtlaufzeit und der Lichtintensitätsschwächung an den Meßlichtweg (62) angepaßt ist, wobei das Meßlicht zwischen einem den Meßlichtweg (62) einschließenden Probenlichtweg (63) und dem Referenzlichtweg (56) optisch umschaltbar ist.

26. Vorrichtung nach einem der Ansprüche 15 bis 25, **dadurch gekennzeichnet**, daß die Meßeinrichtung (38) einen ersten und einen zweiten mit konstanter Differenz oszillierenden Meßfrequenzgenerator (40,41) aufweist, wobei die Differenzfrequenz zwischen den Frequenzen des ersten Frequenzgenerators (40) und des zweiten Frequenzgenerators (41) von einem Differenzfrequenzgenerator (66) vorgegeben und die Frequenz des zweiten Meßfrequenzgenerators (41) auf Basis der vorgegebenen Differenzfrequenz so geregelt wird, daß der zweite Frequenzgenerator (41) Frequenzschwankungen des ersten Frequenzgenerators (40) mit konstanter Differenzfrequenz folgt.

## Claims

1. Method for the analytical determination of the concentration of an analyte in a biological sample, in which
in a detection step light is irradiated by means of irradiation means irradiated into the sample at an irradiation site as primary light through an interface bounding the biological sample and light emerging out of the biological sample through an interface bounding the sample is detected as secondary light at a detection site at a predetermined measuring distance by means of detection means, in order to determine a measurable physical property of the light as a measurement variable which varies due to the interaction with the biological sample, which property correlates with the concentration of the analyte in the biological sample, and
in an evaluation step the analyte concentration is determined on the basis of the measurement variable measured in the detection step,
**characterized in that** in the detection step
(a) frequency-domain spectroscopic measurements are carried out with at least two different measurement light paths between the irradiation site and the detection site and without movement of the light irradiation means or of the detection means
(b) each of the frequency-domain spectroscopic measurements is carried out at at least two different wavelengths of light and
(c) in each of the frequency-domain spectroscopic measurements the phase shift of the secondary light compared with the primary light is determined as well as an intensity measurement variable.

2. Method according to claim 1, **characterized in that** in the evaluation step from the measured values of the frequency-domain spectroscopic measurements an absorption parameter which is a measure of the absorption of the light in the biological sample and/or a scattering parameter which is a measure of the scattering of the light in the biological sample are determined as measurement quantities independent of the respective other parameter, wherein the analyte concencentration is derived from at least one of the parameters.

3. Method according to any one of the preceding claims, **characterized in that** the intensity measurement variable is the AC intensity component of the secondary light.

4. Method according to any one of the preceding claims, **characterized in that** the intensity measurement variable is the DC intensity component of the secondary light.

5. Method according to any one of the preceding claims, **characterized in that** during the frequency-domain spectroscopic measurements light emerging at different detection sites is detected with a plurality of detection means, wherein the detection sites have different measuring distances from at least one irradiation site, thereby to set movement-free the different measurement light paths.

6. Method according to any one of the preceding claims, **characterized in that** during the frequency-domain spectroscopic measurements the primary light is modulated with different modulation frequencies for the movement-free setting of different measurement light paths with an identical measuring distance between the irradiation site and the detection site.

7. Method according to any one of the preceding claims, **characterized in that** the concentration of the analyte is derived essentially from the absorption parameter, the analyte being in particular water, glucose, bilirubin, cholesterol, triglyceride, urea, uric acid, oxyhaemoglobin, haemoglobin or cytochromoxidase.

8. Method according to any one of the preceding claims, **characterized in that** the biological sample is a biological fluid, in particular blood.

9. Method according to any one of claims 1 to 7, **characterized in that** the biological sample is biological tissue, in particular of the finger pads, the upper abdominal walls, the nail bed, the lip, the tongue or the inner upper arm or muscular tissue or the tissue of the sclerae or subcutaneous adipose tissue.

10. Method according to any one of the preceding claims, **characterized in that** an exogenic substance is added to the biological sample which influences the scattering parameter and/or the absorption parameter in the tissue and the concentration of the exogenic substance is determined as analyte in a partial volume of the tissue.

11. Method according to any one of the preceding claims, **characterized in that** the measurement light paths in the tissue are directed to a predetermined partial volume of the tissue in which the analyte concentration is to be determined.

12. Method according to claim 11, **characterized in that** the partial volume is the capillary base of the skin.

13. Method according to claim 11 or 12, **characterized in that** a reflective surface supported on the tissue surface is provided as a means for adjusting the partial volume.

14. Method according to any one of claims 6 to 13, **characterized in that** the modulation frequency and the measuring distance are coordinated with one another in such a way that the light path passes essentially in the predetermined partial volume of the tissue.

15. Apparatus for carrying out the method according to any one of the preceding claims, with
a measuring head (12) provided for butting against an interface of the biological sample,
a measuring instrument (38) for determining as a measurement variable a physical property of the light which varies due to its interaction with the biological sample (10), which property correlates with the concentration of the analyte in the biological sample (10), with
- irradiation means (42) with a light transmitter (43) for irradiating primary light into the biological sample (10) at an irradiation site (17) through an interface (11) bounding the biological sample (10) and
- detection means (48) with a light receiver (44) for detecting at a detection site (18) secondary light emerging out of the biological sample (10) through an interface (11) bounding the sample and
evaluation means (50) for determining the analyte concentration from the measured value of the measurement variable,
**characterized in that**
the measuring instrument (38)
- includes a frequency generator (40) for controlling the primary light transmitter (3), whereby the primary light is modulated with a high-frequency carrier frequency,
- includes a phase measuring device and an intensity measuring device for determining as measurement variables the phase shift of the secondary light compared with the primary light and an intensity measurement variable of the secondary light and
- is so constructed that the measurement variables can be determined without movement of the irradiation means (42) and of the detection means (48) with at least two different measurement light paths (62, 62') between the irradiation site (17) and the detection site (18) and at least two wavelengths of light in each case.

16. Apparatus according to claim 15, **characterized in that** the light transmitter (43) is activated by the frequency generator (40) via a driver stage (40a) and the driver stage (40a) and the light transmitter (43), as well as, preferably, the frequency generator (40), are arranged in an electromagnetically insulating housing.

17. Apparatus according to any one of claims 15 or 16, **characterized in that** the measuring instrument (38) includes a second frequency generator (41) which is likewise arranged in an electromagnetically insulating housing (52).

18. Apparatus according to any one of claims 15 to 17, **characterized in that** the measuring instrument comprises a plurality of detection means (48a, 48b ...), each with a light receiver (44a, 44b ...), which detect simultaneously light emerging from the biological sample at different detection sites, and the light receivers (44a, 44b ...) are arranged on a common carrier (26).

19. Apparatus according to claim 18, **characterized in that** a temperature measuring element (33) is thermally coupled to the carrier (26) and the output signal of the temperature measuring element (33) is fed to the evaluation means (50) in order to take account of the temperature of the light receivers (44) in the determination of the analyte concentration.

20. Apparatus according to any one of claims 15 to 19, **characterized in that** a high-frequency amplifier is coupled to the output of a light receiver (44) in the measuring instrument and the distance between the light receiver and the high-frequency amplifier connected to its output is minimized.

21. Apparatus according to any one of claims 15 to 20, **characterized in that** the output signal of a light receiver (44) in the measuring instrument is fed to a signal mixer (45), and the signal path (64) between the light receiver (44) and the signal mixer (45) is minimized.

22. Apparatus according to claim 21, **characterized in that** the signal mixer (45) is a FET mixer and the light receiver (44) is coupled directly to the gate of the FET mixer.

23. Apparatus according to claim 21, **characterized in that** the light receiver (44) is a photodiode and the photodiode forms part of the signal mixer (45).

24. Apparatus according to any one of claims 15 to 23, **characterized in that** the measuring head (12) includes a plurality of detection means (48, 48') which detect light emerging from the biological sample (10) via a plurality of sample light paths (63, 63') at different detection sites (18, 18'), wherein the light is optically switchable between the sample light paths (63, 63') onto a common light receiver (44).

25. Apparatus according to any one of claims 15 to 24, **characterized in that** the measuring instrument includes an optical reference light section (56) which is adapted with respect to the light transit time and the light intensity attenuation to the measurement light path (62), wherein the measurement light is optically switchable between a sample light path (63), which includes the measurement light path (62), and the reference light path (56).

26. Apparatus according to any one of claims 15 to 25, **characterized in that** the measuring instrument (38) comprises a first measurement frequency generator (40) and a second measurement frequency generator (41) which oscillate with constant difference, wherein the difference frequency between the frequencies of the first frequency generator (40) and the second frequency generator (41) is predetermined by a difference frequency generator (66) and the frequency of the second measurement frequency generator (41) is controlled on the basis of the predetermined difference frequency in such a way that the second frequency generator (41) follows frequency fluctuations of the first frequency generator (40) with a constant difference frequency.

## Revendications

1. Procédé pour la détermination analytique de la concentration d'un analyte dans un échantillon biologique, dans lequel,
dans une étape de détection, on envoie dans l'échantillon de la lumière, à l'aide de dispositifs d'irradiation, à travers une interface qui limite l'échantillon biologique, en tant que lumière primaire, en un endroit d'irradiation et dans lequel on détecte, en un endroit de détection, à une distance de mesure prédéfinie de la zone d'irradiation, la lumière qui sort de l'échantillon biologique à travers une interface qui limite l'échantillon biologique, en tant que lumière secondaire, à l'aide de dispositifs de détection, afin de déterminer une propriété physique de la lumière, mesurable et qui change par l'interaction avec l'échantillon biologique, en tant que grandeur mesurée, qui présente une corrélation avec la concentration de l'analyte dans l'échantillon biologique et
dans lequel on détermine, dans une étape d'évaluation, la concentration en analyte sur base de la grandeur mesurée dans l'étape de détection,
caractérisé en ce qu'au cours de l'étape de détection,
(a) on réalise, sans mouvement des dispositifs d'irradiation de la lumière ou des dispositifs de détection, des mesures de spectroscopie fréquentielle, avec au moins deux parcours optiques de mesure différents entre l'endroit d'irradiation et l'endroit de détection,
(b) on réalise chacune des mesures de spectroscopie fréquentielle à au moins deux longueurs d'onde différentes de la lumière, et
(c) lors des mesures de spectroscopie fréquentielle, on détermine à chaque fois le déplacement des phases de la lumière secondaire par rapport à la lumière primaire, ainsi qu'une grandeur mesurée d'intensité.

2. Procédé selon la revendication 1, caractérisé en ce qu'au cours de l'étape d'évaluation, on détermine, à partir des grandeurs mesurées des mesures de spectroscopie fréquentielle, un paramètre d'absorption, qui est une mesure de l'absorption de la lumière dans l'échantillon biologique et/ou un paramètre de dispersion, qui est une mesure de la dispersion de la lumière dans l'échantillon biologique, en tant que grandeur mesurée indépendante des autres paramètres respectifs, en déduisant la concentration en analyte à partir d'au moins un des paramètres.

3. Procédé selon l'une quelconque des revendications précédentes, caractérisé en ce que la grandeur mesurée d'intensité est la composante d'intensité AC de la lumière secondaire.

4. Procédé selon l'une quelconque des revendications précédentes, caractérisé en ce que la grandeur mesurée d'intensité est la composante d'intensité DC de la lumière secondaire.

5. Procédé selon l'une quelconque des revendications précédentes, caractérisé en ce que, lors des mesures de spectroscopie fréquentielle, pour le réglage immobile des différents parcours optiques de mesure, on capte la lumière sortant en différents endroits de détection à l'aide de plusieurs dispositifs de détection, les endroits de détection présentant des distances de mesure différentes par rapport à au moins un endroit d'irradiation.

6. Procédé selon l'une quelconque des revendications précédentes, caractérisé en ce que, lors des mesures de spectroscopie fréquentielle pour le réglage immobile des différents parcours optiques avec une même distance de mesure entre l'endroit d'irradiation et l'endroit de détection, la lumière primaire est modulée par différentes fréquences de modulation.

7. Procédé selon l'une quelconque des revendications précédentes, caractérisé en ce que la concentration de l'analyte est essentiellement déduite du paramètre d'absorption, l'analyte étant en particulier de l'eau, du glucose, de la bilirubine, du cholestérol, des triglycérides, de l'urée, de l'acide urique, de l'oxyhémoglobine, de l'hémoglobine ou une cytochrome-oxydase.

8. Procédé selon l'une quelconque des revendications précédentes, caractérisé en ce que l'échantillon biologique est un liquide biologique, en particulier du sang.

9. Procédé selon l'une quelconque des revendications 1 à 7, caractérisé en ce que l'échantillon biologique est un tissu biologique, en particulier de la pulpe de doigts, du tissu du ventre, de la matrice des ongles, des lèvres, de la langue, de bras intérieur, des muscles, des sclérotiques ou du tissu graisseux sous-cutané.

10. Procédé selon l'une quelconque des revendications précédentes, caractérisé en ce qu'on ajoute, à l'échantillon biologique, une substance exogène, qui influence les paramètres de dispersion et/ou d'absorption dans le tissu et en ce que la concentration de la substance exogène est déterminée en tant qu'analyte dans un volume partiel du tissu.

11. Procédé selon l'une quelconque des revendications précédentes, caractérisé en ce que les parcours optiques de mesure dans le tissu sont réglés sur un volume partiel prédéfini du tissu dans lequel on veut déterminer la concentration en analyte.

12. Procédé selon la revendication 11, caractérisé en ce que le volume partiel est le lit capillaire de la peau.

13. Procédé selon la revendication 11 ou 12, caractérisé en ce qu'on a prévu, comme dispositif pour le réglage du volume partiel, une surface réfléchissante située sur la surface de tissu.

14. Procédé selon l'une quelconque des revendications 6 à 13, caractérisé en ce que la fréquence de modulation et la distance de mesure sont réglées l'une par rapport à l'autre de telle manière que le parcours optique passe essentiellement dans le volume partiel prédéfini du tissu.

15. Dispositif pour la réalisation du procédé selon l'une quelconque des revendications précédentes, présentant
une tête de mesure (12) prévue pour être placée au niveau de l'interface de l'échantillon biologique
un dispositif de mesure (38) pour la détermination d'une propriété physique de la lumière, qui varie par l'interaction de la lumière avec l'échantillon biologique (10) en tant que grandeur mesurée, qui présente une corrélation avec la concentration de l'analyte dans l'échantillon biologique (10), présentant
- des dispositifs d'irradiation (42) avec un émetteur de lumière (43) pour irradier la lumière primaire dans l'échantillon biologique (10) en un endroit d'irradiation (17) à travers une interface (11) limitant l'échantillon biologique (10), et
- des dispositifs de détection (48) comprenant un récepteur de lumière (44) pour la détection de la lumière secondaire sortant de l'échantillon biologique (10) en un endroit de détection (18) à travers une interface (11) limitant ledit échantillon, et
des dispositifs d'évaluation (50) pour la détermination de la concentration en analyte à partir de la valeur mesurée de la grandeur mesurée, caractérisé en ce que le dispositif de mesure (38)
- comprend un générateur de fréquences (40) pour la commande de l'émetteur de lumière primaire (3), la lumière primaire étant modulée par une fréquence porteuse à fréquence élevée,
- comprend un dispositif de mesure des phases et un dispositif de mesure de l'intensité, afin de déterminer le déplacement des phases de la lumière secondaire par rapport à la lumière primaire et une grandeur de mesure de l'intensité de la lumière secondaire comme grandeurs de mesure, et
- est exécuté de telle manière que les grandeurs de mesure peuvent être déterminées sans mouvement du dispositif d'irradiation (42) ni du dispositif de détection (48) à au moins deux parcours optiques de mesure différents (62, 62') entre l'endroit d'irradiation (17) et l'endroit de détection (18) à au moins chaque fois deux longueurs d'onde différentes de la lumière.

16. Dispositif selon la revendication 15, caractérisé en ce que l'émetteur de lumière (43) est commandé, via une étape d'attaque (40a), par le générateur de fréquences (40) et en ce que l'étape d'attaque (40a) et l'émetteur de lumière (43), de préférence également le générateur de fréquences (40), sont disposés dans un bâti isolé électromagnétiquement.

17. Dispositif selon l'une quelconque des revendications 15 à 16, caractérisé en ce que le dispositif de mesure (38) comprend un deuxième générateur de fréquences (41) qui est également disposé dans un bâti (52) isolé électromagnétiquement.

18. Dispositif selon l'une quelconque des revendications 15 à 17, caractérisé en ce que le dispositif de mesure présente une multitude de dispositifs de détection (48a, 48b, ...) avec chaque fois un récepteur de lumière (44a, 44b, ...), qui détectent simultanément la lumière sortant de l'échantillon biologique en différents endroits de détection et en ce que les récepteurs de lumière (44a, 44b, ...) sont disposés sur une pièce support (26) commune.

19. Dispositif selon la revendication 18, caractérisé en ce que la pièce support (26) est accouplée thermiquement à un élément de mesure de la température (33) dont le signal de sortie est acheminé vers les dispositifs d'évaluation (50) afin de tenir compte de la température des récepteurs de lumière (44) lors de la détermination de la concentration en analyte.

20. Dispositif selon l'une quelconque des revendications 15 à 19, caractérisé en ce qu'un amplificateur de hautes fréquences est placé en aval d'un récepteur de lumière (44) dans le dispositif de mesure et en ce que la distance entre le récepteur de lumière et l'amplificateur de hautes fréquences placé en aval est minimisée.

21. Dispositif selon l'une quelconque des revendications 15 à 20, caractérisé en ce que le signal de sortie d'un récepteur de lumière (44) dans le dispositif de mesure est acheminé vers le dispositif de mixage des signaux (45), et en ce que le parcours du signal (64) entre le récepteur de lumière (44) et le dispositif de mixage des signaux (45) est minimisé.

22. Dispositif selon la revendication 21, caractérisé en ce que le dispositif de mixage des signaux (45) est un dispositif de mixage FET et en ce que le récepteur de lumière (44) est directement accouplé à la porte de circuit logique du dispositif de mixage FET.

23. Dispositif selon la revendication 21, caractérisé en ce que le récepteur de lumière (44) est une photodiode et en ce que la photodiode est une composante du dispositif de mixage des signaux (45).

24. Dispositif selon l'une quelconque des revendications 15 à 23, caractérisé en ce que la tête de mesure (12) comprend plusieurs dispositifs de détection (48, 48') qui détectent, via plusieurs parcours optiques de l'échantillon (63, 63') en différents endroits de détection (18, 18'), la lumière sortant de l'échantillon biologique (10), la lumière pouvant être commutée optiquement entre les parcours optiques (63, 63') de l'échantillon sur un récepteur de lumière (44) commun.

25. Dispositif selon l'une quelconque des revendications 15 à 24, caractérisé en ce que le dispositif de mesure comprend une zone (56) optique de référence, qui est adaptée au parcours optique (62) de mesure, eu égard à la durée de parcours de la lumière et l'affaiblissement de l'intensité de la lumière, la lumière de mesure pouvant être commutée optiquement entre un parcours optique de l'échantillon (63), comprenant le parcours optique de mesure (62), et le parcours optique de référence (56).

26. Dispositif selon l'une quelconque des revendications 15 à 25, caractérisé en ce que le dispositif de mesure (38) présente un premier et un deuxième générateur de fréquences de mesure (40, 41) oscillant avec une différence constante, la fréquence différentielle entre les fréquences du premier générateur de fréquences (40) et du deuxième générateur de fréquences (41) étant indiquée par un générateur de fréquences différentielles (66) et la fréquence du deuxième générateur de fréquences de mesure (41) étant réglée, sur base de la fréquence différentielle indiquée, de telle manière que le deuxième générateur de fréquences (41) suit les oscillations de fréquences du premier générateur de fréquences (40) avec une fréquence différentielle constante.
